# EUROPÄISCHE PATENTSCHRIFT

(11) **EP 2 648 805 B1**
(45) Veröffentlichungstag und Bekanntmachung des Hinweises auf die Patenterteilung: **10.08.2016**
(21) Anmeldenummer: 11787666.4
(22) Anmeldetag: 17.11.2011
(51) Int. Cl.: A61N 5/10

(54) **BESTRAHLUNGSPHANTOM**
RADIATION PHANTOM
FANTÔME D'IRRADIATION

(30) Priorität: 08.12.2010 DE 102010061121
(43) Veröffentlichungstag der Anmeldung: 16.10.2013
(73) Patentinhaber: GSI Helmholtzzentrum für Schwerionenforschung GmbH, 64291 Darmstadt (DE)
(72) Erfinder: SCHUBERT, Elko, 64823 Groß-Umstadt (DE); STEIDL, Peter, 64285 Darmstadt (DE); RICHTER, Daniel, 64888 Darmstadt (DE); SCHUY, Christoph, 63225 Langen (DE); BERT, Christoph, 63741 Aschaffenburg (DE)
(86) Internationale Anmeldenummer: PCT/EP2011/070337
(87) Internationale Veröffentlichungsnummer: WO 2012/076311

(56) Entgegenhaltungen:
- US-A1- 2005 211 889
- US-A1- 2007 140 413
- US-A1- 2008 011 946
- US-A1- 2008 298 540
- US-A1- 2009 110 140
- BRUSASCO C ET AL: "A dosimetry system for fast measurement of 3D depth-dose profiles in charged-particle tumor therapy with scanning techniques", NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL, Bd. 168, Nr. 4, 1. August 2000 (2000-08-01), Seiten 578-592, XP004206343, ISSN: 0168-583X, DOI: 10.1016/S0168-583X(00)00058-6

## Beschreibung

Die Erfindung betrifft eine Bestrahlungsphantomvorrichtung, insbesondere eine Bestrahlungsphantomvorrichtung zur Validierung eines Bestrahlungsvorgangs, bevorzugt zur Validierung einer Bestrahlungsplanung, die zumindest eine Bewegungsvorrichtung zur Bewegung von zumindest einem ersten Teilbereich der Bestrahlungsphantomvorrichtung, insbesondere relativ zu zumindest einem zweiten Teilbereich der Bestrahlungsphantomvorrichtung aufweist. Weiterhin betrifft die Erfindung ein Verfahren zur Validierung eines Bestrahlungsvorgangs, insbesondere zur Validierung einer Bestrahlungsplanung, wobei der Bestrahlungsvorgang an einer Bestrahlungsphantomvorrichtung erfolgt, die zumindest eine Bewegungsvorrichtung zur Bewegung von zumindest einem ersten Teilbereich der Bestrahlungsphantomvorrichtung, insbesondere relativ zu zumindest einem zweiten Teilbereich der Bestrahlungsphantomvorrichtung aufweist.

Die Bestrahlung von Gegenständen mit ionisierender Strahlung macht es möglich, diese auch im Inneren mit einer bestimmten Strahlungsdosis beaufschlagen zu können. Je nach verwendeter Strahlung und Aufgabenstellung erfolgt dabei eine mehr oder weniger orts-unaufgelöste Bestrahlung (d. h., der Körper wird im Wesentlichen über seinen gesamten Volumenbereich hinweg mit einer im Wesentlichen gleichen Strahlungsdosis beaufschlagt), flächenaufgelöste Bestrahlung (d. h., es erfolgt eine strukturierte Beaufschlagung in Abhängigkeit von den x- bzw. y-Koordinaten in einer Ebene, jedoch keine strukturierte Bestrahlung in der Tiefe bzw. längs der z-Richtung) oder aber auch eine dreidimensional strukturierte Bestrahlung (d. h., eine Bestrahlung mit einer Ortsauflösung in allen drei Raumrichtungen).

Als ein Anwendungsbeispiel unter vielen für derartige Bestrahlungen sind Bestrahlungen im medizinischen Bereich zu nennen. Das Spektrum für einen Einsatz ionisierender Strahlung in der Medizin reicht zwischenzeitlich über einen weiten Bereich. Beispielsweise haben sich seit vielen Jahrzehnten Röntgenaufnahmen als Diagnoseverfahren etabliert, bei denen ein Patient mit einer im Wesentlichen unstrukturierten Strahlung beaufschlagt wird. Je nach durchstrahlter Gewebeart (beispielsweise Weichgewebe, Knochen, Lufthohlräume und dergleichen) kommt es zu einer unterschiedlich starken Abschwächung der Röntgenstrahlung und damit zu einer unterschiedlichen Schwärzung des hinter dem Patienten befindlichen Röntgenfilms. Ein Anwendungsbeispiel für eine dreidimensional strukturierte Bestrahlung ist die Behandlung von Tumoren mit Teilchenstrahlung. Hierzu werden zum Teil Photonen und Elektronen verwendet. In den letzten Jahren hat sich jedoch auch die Therapie von Tumoren mit Schwerionenstrahlung sehr stark entwickelt, da Schwerionenstrahlung aufgrund ihres ausgeprägten Bragg-Peaks eine hervorragende Möglichkeit darstellt, eine genaue Strukturierung in der Tiefe (in z-Richtung; parallel zum Teilchenstrahl) im Millimeterbereich zu realisieren.

Insbesondere bei der Krebstherapie ist es erforderlich, in einem innerhalb des Körpers befindlichen Volumenbereich (nämlich am Ort des zu therapierenden Tumors) eine bestimmte, vergleichsweise hohe und zellschädigende Dosis einzubringen, wohingegen das sonstige Gewebe des Patienten eine möglichst geringe Strahlendosis erfahren sollte. Dies ist insbesondere für kritische Gewebebereiche (oftmals als OAR für "Organ At Risk" bezeichnet) der Fall, bei denen bestimmte (in der Regel vergleichsweise niedrig liegende) Maximalstrahlungsdosen unter keinen Umständen überschritten werden dürfen. Derartige kritische Gewebebereiche sind beispielsweise Nervengewebe, Blutgefäße, bestimmte innere Organe und dergleichen.

Um eine derartige dreidimensional strukturierte Bestrahlung einbringen zu können muss die Bestrahlungsvorrichtung geeignet justiert werden (beispielsweise hinsichtlich Strahlführung, Teilchenenergie und dergleichen). Da sich zwischenzeitlich sogenannte Scan-Verfahren etabliert haben, bei denen ein bleistiftdünner Teilchenstrahl (ein so genannter "pencil-beam") das zu therapierende Gewebe zeilen-, spalten- und scheibenweise sukzessive nacheinander abfährt, ist gegebenenfalls auch ein zeitlich hintereinander anzuwendender Parametersatz zur (zeitlich variierenden) Ansteuerung der Bestrahlungsvorrichtung erforderlich. Hierzu sind in der Praxis umfangreiche Berechnungen erforderlich, wobei auch bestimmte Annahmen und Erfahrungswerte (die nicht notwendigerweise exakt zutreffend sind) in die Berechnung eingehen. Um sicher zu sein, dass die gewonnenen Parameter zutreffend sind, werden bereits seit geraumer Zeit sogenannte Bestrahlungsphantome verwendet. Diese werden zu Überprüfungszwecken anstelle des Patienten mit der Bestrahlung beaufschlagt. Erst wenn das Bestrahlungsergebnis zufriedenstellend ist, wird die errechnete Bestrahlungsplanung tatsächlich in den Patienten eingebracht.

Besondere Herausforderungen - sowohl bezüglich des Bestrahlungsphantoms, als auch bezüglich der Anforderungen an die Berechnung einer Bestrahlungsplanung - ergeben sich, wenn auch Bewegungen berücksichtigt werden müssen. Dies ist beispielsweise dann der Fall, wenn sich der Tumor während der Therapie merklich bewegt. Dies ist beispielsweise dann der Fall, wenn sich der Tumor in bzw. benachbart zur Lunge, in bzw. benachbart zum Herzen oder am bzw. benachbart zum Darm befindet.

Zwischenzeitlich wurden unterschiedlichste Verfahren vorgeschlagen, um auch bei derartigen Rahmenbedingungen eine Bestrahlungsplanung errechnen zu können. Eine Zusammenfassung findet sich beispielsweise in der Veröffentlichung "Special report: workshop on 4D-treatment planning in actively scanned particle therapy - recommendation, technical challenges, and future research directions" von A. Knopf, C. Bert, E. Heath, S. Nill, K. Kraus, D. Richter, E. Hug, E. Pedroni, S. Safai, F. Albertini, S. Zenklusen, D. Boye, M. Söhn, N. Soukup, B. Sobotta und A. Lomax in Med. Phys. 37 (9), September 2010, Seite 4.608 - 4.614.

Obgleich die bereits beschriebenen Berechnungsverfahren und Bestrahlungsvorrichtungen sehr vielversprechend sind, ist es nach wie vor ein Problem, deren Güte vorab zu überprüfen, bevor ein Tier oder ein Mensch mit der geplanten Dosis gemäß der Bestrahlungsplanung beaufschlagt wird.

So existieren zwar bislang unterschiedlichste Bestrahlungsphantome, die jedoch oftmals nicht beweglich, insbesondere nicht in sich beweglich, sind. Sofern Bewegungen simuliert werden können, erweisen sich die dann üblicherweise verwendeten Metallbauteile als problematisch, da sie entweder den eigentlichen Bestrahlungsvorgang oder aber zumindest die Messvorgänge erschweren oder sogar unmöglich machen bzw. sich die dabei gewonnenen Daten bis hin zur Unbrauchbarkeit verschlechtern können. Darüber hinaus erweisen sich die bei bislang bekannten Bestrahlungsphantomen darstellbaren Bewegungen als oftmals unzureichend. Beispielsweise ist bei vielen bekannten Bestrahlungsphantomen lediglich eine 1 D-Bewegung darstellbar.

Die Patentanmeldung US-A1-2007/140413 offenbart eine Bestrahlungsphantomvorrichtung gemäß dem Oberbegriff des Anspruchs 1.

Es ist daher die Aufgabe der Erfindung eine Bestrahlungsphantomvorrichtung vorzuschlagen, die gegenüber bekannten Bestrahlungsphantomvorrichtungen verbessert ist. Eine weitere Aufgabe der Erfindung besteht darin, ein Verfahren zur Validierung eines Bestrahlungsvorgangs vorzuschlagen, welches gegenüber bekannten Verfahren zur Validierung von Bestrahlungsvorgängen verbessert ist.

Die Erfindung löst die Aufgabe. Die Erfindung wird in dem beigefügten Anspruchssatz definiert

Es wird vorgeschlagen, eine Bestrahlungsphantomvorrichtung, die zumindest eine Bewegungsvorrichtung zur Bewegung von zumindest einem ersten Teilbereich der Bestrahlungsphantomvorrichtung aufweist, derart auszubilden, dass die Bestrahlungsphantomvorrichtung zumindest zeitweise und/oder zumindest bereichsweise bestrahlungsphantomvorbildkonforme Bestrahlungseigenschaften aufweist. Bei der Bestrahlungsphantomvorrichtung kann es sich insbesondere um eine Bestrahlungsphantomvorrichtung zur Validierung eines Bestrahlungsvorgangs handeln. Bevorzugt kann es sich bei der Bestrahlungsphantomvorrichtung um eine Bestrahlungsphantomvorrichtung zur Validierung einer Bestrahlungsplanung handeln. Die Bewegung von zumindest einem ersten Teilbereich der Bestrahlungsphantomvorrichtung kann vorzugsweise relativ zu zumindest einem zweiten Teilbereich der Bestrahlungsphantomvorrichtung erfolgen. Bei der Bestrahlungsphantomvorrichtung kann es sich grundsätzlich um eine beliebige Art von Bestrahlungsphantom handeln. So ist in diesem Zusammenhang an biologische Proben wie beispielsweise Zellkulturen oder dergleichen zu denken. Die Zellkulturen können dabei durchaus ortsaufgelöst verwendet werden, beispielsweise indem eine zweidimensionale oder dreidimensionale Anordnung (Array) aus einzelnen, voneinander getrennten Zellkulturproben verwendet wird. Durch Untersuchung, welcher Probenteil bzw. welche Teilproben wie stark geschädigt wurde, kann dementsprechend eine ortsaufgelöste Messung durchgeführt werden. Denkbar - und in der Regel bevorzugt - sind jedoch sonstige Detektorentypen, wie beispielsweise Filmdetektoren (wobei typischerweise der Schwärzungsgrad der entsprechenden Filme als Messwert für den Strahlungseintrag verwendet wird), Halbleiterdetektoren, lonisationsdetektoren und dergleichen. Selbstverständlich können auch diese und weitere Detektorentypen zweidimensional oder dreidimensional ortsauflösend genutzt werden. Auch eine Kombination aus derartigen "sonstigen" Detektorentypen und "biologischen" Detektoren kann sich als vorteilhaft erweisen. Vorzugsweise weist die Bestrahlungsphantomvorrichtung neben den bereits genannten vorteilhaften Detektoreinheiten auch "passive" Bereiche auf, die beispielsweise das Vorhandensein von Gewebe, sonstigen Materialabschnitten und dergleichen simulieren können. Die zumindest eine Bewegungsvorrichtung kann ebenfalls in an sich beliebiger Weise aufgebaut sein. Auch die Antriebsart (beispielsweise elektrisch, pneumatisch, hydraulisch und dergleichen) ist grundsätzlich beliebig wählbar. Möglich ist es, dass die Bewegungseinrichtung beispielsweise durch externe Steuersignale angesteuert werden kann. Dies ist vorzugsweise in einem weiten Bereich möglich. Insbesondere ist es denkbar, dass die Bewegung einfach ein- bzw. ausgeschaltet wird. Denkbar ist es aber auch, dass die Geschwindigkeit einer Bewegung verstellbar ist. Weiterhin ist es vorteilhaft, wenn im Wesentlichen beliebige Bewegungen mit Wesentlichen beliebigen Geschwindigkeiten (wobei die obere Grenze der Geschwindigkeiten insbesondere durch das Bestrahlungsphantomvorbild, gegebenenfalls zuzüglich eines Sicherheitszuschlags, definiert sein kann) durchgeführt werden können. Vorzugsweise ist die Bewegungsvorrichtung derart ausgeführt, dass diese neben einem Standard-Bewegungszyklus auch Nicht-Standard-Bewegungszyklen zulässt und/oder diese den Standard-Bewegungszyklus modifizieren kann. Rein beispielsweise kann es sich um die Atmung eines Patienten handeln. Der Bewegungszyklus ist typischerweise für einen einzelnen Patienten relativ konstant. Dennoch kann es vorkommen, dass der Patient beispielsweise während der eigentlichen Bestrahlung in Folge von Nervosität schneller und/oder tiefer (selbstverständlich auch langsamer und/oder flacher) atmet. Dementsprechend ist eine Anpassung des Standard-Bewegungszyklusses sinnvoll. Weiterhin kann es sich als vorteilhaft erweisen, wenn gewissermaßen Bewegungen mit unterschiedlichen Parametern zueinander überlagert werden. Dies kann sowohl durch eine entsprechende Ansteuerung einer einzelnen Bewegungsvorrichtung, als auch durch die Verwendung mehrerer Bewegungsvorrichtungen erfolgen. Derartige Parameter sind dabei insbesondere die jeweilige Bewegungsphase, die Periode und/oder die Amplitude. Rein beispielsweise soll hier ein Tumor, welcher sich an einem Lungenflügel befindet, genannt sein. Der Brustkorb (Thorax) bewegt sich beispielsweise mit einer bestimmten Amplitude A1 und Phase B1. Der eigentliche Tumor weist dagegen eine Bewegung mit einer davon abweichenden Amplitude A2 und Phase B2 auf. Die beiden Bewegungen mit den jeweiligen Amplitude A1 und A2 sowie den jeweiligen Phasen B1 und B2 überlagern sich dabei und erzeugen ein relativ komplexes Gesamt-Bewegungsmuster. Darüber hinaus ist es auch möglich, dass der Patient während der Bestrahlung irreguläre Bewegungen, wie sie beispielsweise durch Husten, Schluckauf oder dergleichen hervorgerufen werden können, durchführt. Dementsprechend ist es vorteilhaft, wenn auch die Bestrahlungsphantomvorrichtung derartige irreguläre Bewegungen simulieren kann. Sämtliche der genannten Bewegungen (insbesondere zyklisch wiederkehrende Bewegungen) können beispielsweise durch "Hardwareaufbauten" (beispielsweise Kurvenscheibengetriebe und dergleichen) zumindest zum Teil erzeugt werden. Bevorzugt ist es jedoch, wenn die Ansteuerung der Bewegungsvorrichtung beispielsweise durch geeignete Stellimpulse auf im Wesentlichen beliebige Art und Weise durchgeführt werden kann. Die Bestrahlungsphantomvorrichtung ist dann besonders universell verwendbar. Bei der Bewegung, die durch zumindest eine Bewegungsvorrichtung durchgeführt wird, kann sich dabei nicht nur um eine Bewegung gegenüber den externen Raumkoordinaten handeln. Besonders vorteilhaft (weil typisch für viele Bestrahlungsvorgänge) ist es, wenn die Bewegung des bewegten ersten Teilbereichs relativ zu zumindest einem zweiten Teilbereich der Bestrahlungsphantomvorrichtung erfolgt. Hier kann es sich beispielsweise um die Bewegung eines im Lungengewebe sitzenden Tumors relativ zu den meisten Körperbereichen (wie beispielsweise der Wirbelsäule und dergleichen) handeln. Die (Relativ-)Bewegung kann sich dabei auf eine, zwei, bevorzugt drei Raumrichtungen (Translationsfreiheitsgrade) beziehen. Zusätzlich oder alternativ kann es sich auch um eine, zwei oder drei Drehbewegungen handeln (Rotationsfreiheitsgrade). Insbesondere kann darüber hinaus auch sinnvollerweise eine Stauchung und/oder Dehnung von Gewebe simuliert werden. Aber auch die Vergrößerung bzw. Verkleinerung von Hohlräumen, welche sich insbesondere zwischen zumindest einem ersten Teilbereich und zumindest einem zweiten Teilbereich befinden, kann sich bei der Bestrahlungsphantomvorrichtung als vorteilhaft erweisen, da auch dies in der Realität (beispielsweise bei Lungentumoren) auftreten kann. Selbstverständlich sind auch beliebige Mischformen der bereits erwähnten (sowie weiterer) Bewegungsformen denkbar. Unter dem Begriff "bestrahlungsphantomvorbildkonform" ist insbesondere eine "Konformität" des Bestrahlungsphantoms mit seinem "Vorbild" zu verstehen. Das Vorbild ist dabei in aller Regel die Vorrichtung, der Gegenstand bzw. auch das Tier oder die Person, die mit der Bestrahlungsphantomvorrichtung "simuliert" werden soll. Die "Konformität" bezieht sich dabei insbesondere auf die relevanten Eigenschaften und bedeutet in der Regel, dass die durch diese Eigenschaften bedingten Auswirkungen zumindest ähnlich, vorzugsweise im Wesentlichen gleichartig sind. Vorliegend ist unter einer derartigen Eigenschaft insbesondere der Einfluss auf eingebrachte Strahlung ("Bestrahlungseigenschaft") zu verstehen. Unter einer "bestrahlungsphantomvorbildkonformen Bestrahlungseigenschaft" ist insbesondere eine Eigenschaft der Bestrahlungsphantomvorrichtung gegenüber der eingebrachten Strahlung zu verstehen, so dass diese in Bezug auf ihre Auswirkungen auf die eingebrachte Strahlung dem Vorbild, welches durch die Bestrahlungsphantomvorrichtung simuliert werden soll, möglichst nahe kommt. Dies kann sich insbesondere auf eine besonders gute qualitative und/oder quantitative Annäherung beziehen. Liegt eine (weitgehende bzw. ausreichende) bestrahlungsphantomvorbildkonformität vor, so ist es insbesondere möglich, dass das Bestrahlungsphantomvorrichtung Eigenschaften aufweist, die eine Absolutdosimetrie mit einem lonenstrahl und/oder mit einer sonstigen Art der Bestrahlung ermöglicht. Bei der Strahlung kann es sich insbesondere um ionisierende Strahlung, wie insbesondere Gammastrahlung, Röntgenstrahlung sowie Teilchenstrahlung (Leptonen und Hadronen, insbesondere Elektronen, Positronen, Protonen, Heliumionen, Kohlenstoffionen, Sauerstoffionen, Stickstoffionen, Neonionen) handeln. Die Vorbildkonformität der Bestrahlungsphantomvorrichtung bezieht sich dabei insbesondere im Falle von "Therapiestrahlung" (also beispielsweise Ionenstrahlung bzw. Schwerionenstrahlung) bevorzugt auf die Konformität im Bereich einer Detektoreinrichtung (die nicht notwendigerweise innerhalb der Bestrahlungsphantomvorrichtung liegen muss) und/oder eines proximal vor der Detektoreinrichtung liegenden Bereichs. Insbesondere im Falle von "Messstrahlung" (beispielsweise Röntgenstrahlung, CT-Strahlung und dergleichen) bezieht sich die Vorbildkonformität der Bestrahlungsphantomvorrichtung bevorzugt auf die Konformität im Bereich einer Detektoreinrichtung und/oder auf die Konformität in einem Bereich in Form einer axial liegenden Scheibe und/oder (speziell im Fall einer Kegelstrahlgeometrie) auf die Konformität in einem Kegel(stumpf)artigen Bereich. Denn ein solche (kritischen) Bereiche sind typischerweise der Gegenstand der Untersuchungen. Verfälschungen in hiervon beabstandeten Bereichen haben typischerweise keinen, keinen messbaren, keinen signifikanten keinen relevanten und/oder einen vergleichsweise geringen Einfluss auf die Strahlungsmessung im Bereich der Detektoreinrichtung und/oder des Zielvolumenbereichs. Dementsprechend können durch eine gegebenenfalls einfache und/oder kostengünstigere Bauweise der Bestrahlungsphantomvorrichtung in entfernteren Bereichen Kosten eingespart werden bzw. die technische Funktionalität der Bestrahlungsphantomvorrichtung erhöht werden.

Vorzugsweise ist die Bestrahlungsphantomvorrichtung derart ausgebildet, dass diese zumindest zeitweise und/oder zumindest bereichsweise bestrahlungsphantomvorbildkonforme Behandlungsstrahleigenschaften und/oder zumindest zeitweise und/oder zumindest bereichsweise bestrahlungsphantomvorbildkonforme Messstrahleigenschaften aufweist. Die bestrahlungsphantomvorbildkonformen Bestrahlungsstrahleigenschaften beziehen sich insbesondere auf die Eigenschaften der Bestrahlungsphantomvorrichtung gegenüber dem eigentlichen Behandlungsstrahl. Hierbei handelt es sich in der Regel um einen Teilchenstrahl, wie insbesondere um einen Elektronenstrahl, einen Positronenstrahl, einen Protonenstrahl, einen Heliumionenstrahl, einen Kohlenstoffionenstrahl, einen Sauerstoffionenstrahl, einen Stickstoffionenstrahl, einen sonstigen lonenstrahl und/oder einen sonstigen Schwerionenstrahl. Zum Teil werden aber auch Photonen verwendet wie insbesondere bei der sogenannten IMRT (für Intensity Modulated Radiation Therapy = Intensitätsmodulierte Photonentherapie), die sich als so genannte IMPT (für Intensity Modulated Particle Therapy = intensitätsmodulierte Partikeltherapie) zwischenzeitlich auch bei Schwerionen- oder Protonentherapie etabliert hat. Bei den bestrahlungsphantomvorbildkonformen Messstrahleigenschaften handelt es sich insbesondere um die Eigenschaften der Bestrahlungsphantomvorrichtung gegenüber Messstrahlung, welche insbesondere zu Überprüfungszwecken (wie beispielsweise zur Lageüberprüfung des Tumorbereichs und/oder sonstiger Materialbereiche bzw. Gewebebereiche) eingesetzt wird. Hierbei kann es sich insbesondere um klassische Röntgenstrahlen (beispielsweise offene Felder bei <250 keV) handeln. Gegebenenfalls kann es sich aber auch um Kernspintomographieverfahren und/oder Computertomographieverfahren (insbesondere kV-, MV- und/oder Cone-Beam-Computertomographieverfahren) und/oder sonstige, speziell zur Bildgebung eingesetzte Strahlung handeln. Selbstverständlich sind auch weitere Verfahren denkbar, welche auf Messstrahlung, insbesondere auf durchdringender Messstrahlung, besonders bevorzugt auf ionisierender Messstrahlung beruhen. Üblicherweise erweist sich die Verwendung von derartiger Messstrahlung als vorteilhaft, weil beispielsweise durch die dadurch gewonnenen Daten die zu erwartende lonendosis berechnet werden kann und/oder eine präzise Lagerung der Bestrahlungsphantomvorrichtung (analog zum späteren Patienten) am Bestrahlungsplatz möglich wird.

Insbesondere ist es von Vorteil, wenn bei der Bestrahlungsphantomvorrichtung zumindest zeitweise und/oder zumindest bereichsweise bestrahlungsphantomvorbildkonforme Abschwächungseigenschaften und/oder bestrahlungsphantomvorbildkonforme Sekundärstrahlungsemissionseigenschaften und/oder bestrahlungsphantomvorbildkonforme Streuungseigenschaften vorhanden sind. Insbesondere kann sich dies auf benachbart und/oder proximal (speziell in Bezug auf "Therapiestrahlung", also üblicherweise den "eigentlichen" Bestrahlungsstrahl) und/oder scheibenartig-axial (speziell in Bezug auf CT-Messstrahlung) und/oder kegelartig (speziell in Bezug auf Kegelstrahlen) und/oder fächerartig (speziell in Bezug auf Fächerstrahlen) zu einem Zielvolumenbereich liegende Teilbereiche der Bestrahlungsphantomvorrichtung beziehen. Bei dem Zielvolumenbereich handelt es sich dabei in der Regel um die Lage des "Tumors", wo bei der Bestrahlungsphantomvorrichtung in der Regel zumindest eine Detektorvorrichtung angeordnet ist. Wenn die genannten Eigenschaften (Abschwächungseigenschaften, Sekundärstrahlungsemissionseigenschaften und/oder Streuungseigenschaften) der Bestrahlungsphantomvorrichtung besonders nah an das Bestrahlungsphantomvorbild (an das "Original") herankommen, kann in der Regel die Güte der Bestrahlungsvorgangsvalidierung besonders hoch ausfallen. Bei Abschwächungseigenschaften ist insbesondere an die Abschwächung der verwendeten Strahlung (insbesondere Behandlungsstrahlung und/oder Messstrahlung) zu denken. Bei den Sekundärstrahlungsemissionseigenschaften ist insbesondere an den häufiger auftretenden Effekt zu denken, dass die verwendete Strahlung (insbesondere Messstrahlung und/oder Behandlungsstrahlung) in Interaktion mit bestimmten Materialien Teilchen freisetzen kann, wobei die "Eingangsstrahlung" dabei in aller Regel abgeschwächt wird. Bei den freigesetzten Teilchen kann es sich insbesondere um Neutronen, Elektronen, Positronen, Protonen und dergleichen handeln. Bei Streuungseigenschaften ist nicht nur an eine Winkelablenkung und/oder Winkelaufweitung der genutzten Strahlung (insbesondere Behandlungsstrahlung und/oder Messstrahlung) zu denken, sondern auch an Streuungsmechanismen, bei denen zusätzliche Effekte auftreten, wie beispielsweise die Freisetzung von Teilchen (beispielsweise Compton-Elektronen und dergleichen).

Weiterhin kann es sich als vorteilhaft erweisen, wenn die Bestrahlungsphantomvorrichtung zumindest zeitweise und/oder zumindest bereichsweise bestrahlungsphantomvorbildkonforme Reflektionseigenschaften und/oder bestrahlungsphantomvorbildkonforme Sekundärstrahlungsemissionseigenschaften und/oder bestrahlungsphantomvorbildkonforme Streuungseigenschaften aufweist. Dies gilt insbesondere für benachbart und/oder distal (speziell in Bezug auf "Therapiestrahlung") und/oder scheibenartig-axial (speziell in Bezug auf CT-Messstrahlung) und/oder kegelartig (speziell in Bezug auf Kegelstrahlen) und/oder fächerartig (speziell in Bezug auf Fächerstrahlen) zu einem Zielvolumenbereich liegende Teilbereich der Bestrahlungsphantomvorrichtung. Die genannten Beeinflussungen haben typischerweise einen besonders großen Einfluss auf den bzw. die Zielvolumenbereiche, wenn diese benachbart und/oder distal zum entsprechenden Zielvolumenbereich liegen. Durch eine entsprechende Annäherung der Bestrahlungsphantomvorrichtung an das Bestrahlungsphantomvorbild (also an das "Original") kann abermals eine in der Regel besonders hohe Güte der Validierung des Bestrahlungsvorgangs erzielt werden. Im Übrigen ist bei Reflektionseigenschaften besonders an solche Mechanismen zu denken, bei denen zumindest teilweise ein Einfluss in "rückwärtiger Richtung" auftritt. Hier kann es sich beispielsweise um Streuungsmechanismen und dergleichen handeln, bei denen zumindest ein Teil der Ausgangsstrahlung in einem gewissen Sinne rückwärts gestrahlt wird (also insbesondere um üblicherweise mehr als 90°, mehr als 120°, und/oder mehr als 150° gestreut wird).

Nach der Erfindung ist die Bestrahlungsphantomvorrichtung derart ausgebildet, dass zumindest eine Bewegungsvorrichtung zumindest zeitweise und/oder zumindest bereichsweise eine mehrdimensionale Bewegung durchführen kann, mindest eine 6D-Bewegung. Auf diese Weise kann die Bestrahlungsphantomvorrichtung besonders universell eingesetzt werden. Wie bereits erwähnt, kann es sich bei den Dimensionen insbesondere um Translationsbewegungen und/oder Rotationsbewegungen handeln. Denkbar ist es aber auch, dass die Bestrahlungsphantomvorrichtung zumindest zeitweise und/oder zumindest bereichsweise nicht bewegt wird und/oder lediglich eine eindimensionale Bewegung durchführt. Bei allen genannten Bewegungen bzw. Nichtbewegungen ist selbstverständlich an eine Zeitabhängigkeit der Bewegung zu denken. In Abhängigkeit vom Sprachgebrauch könnte dementsprechend auch von einer Bewegung mit einer um eins erhöhten Dimension gesprochen werden, so dass sich schlussendlich bis zu "siebendimensionale Bewegungen" ergeben können. Weiterhin ist es möglich - und in der Regel bevorzugt - dass eine oder mehrere Bewegungsvorrichtungen zueinander überlagerte Einzel-Bewegungen erzeugen (also beispielsweise eine Überlagerung einer Atembewegung mit einer Herzschlagbewegung und/oder einer sonstigen Bewegung).

Besonders vorteilhaft ist es, wenn die Bestrahlungsphantomvorrichtung zumindest ein Bewegungsübertragungsmittel aufweist, welches zumindest zeitweise und/oder zumindest bereichsweise bestrahlungsphantomvorbildkonforme Bestrahlungseigenschaften aufweist. Die Erfinder haben herausgefunden, dass es sehr schwer bis nahezu unmöglich ist, die Bewegung direkt im Bestrahlungsphantom und insbesondere benachbart zum Zielvolumenbereich zu erzeugen. Dies liegt insbesondere darin begründet, dass im Stand der Technik bekannte Bewegungserzeugungsmittel in aller Regel zumindest zum Teil Metall aufweisen. Metall ist jedoch in aller Regel bezüglich seiner Bestrahlungseigenschaften nicht bestrahlungsphantomvorbildkonform. Allgemeiner formuliert sind (höhere Konzentrationen) von Materialien mit hohen Kernladungszahlen zu vermeiden. Insbesondere sollten die Kernladungszahlen niedriger als die Kernladungszahlen von leichten Metallen (wie beispielsweise Aluminium, Kupfer und/oder Eisen) liegen. Die Erfinder haben jedoch zu ihrer eigenen Überraschung herausgefunden, dass es unproblematisch ist, eine in einer ausreichenden Entfernung erzeugte Bewegung unter Verwendung von Bewegungsübertragungsmitteln an einen Ort, an dem die Bewegung (bzw. die Kraft) vorteilhafterweise ansetzen sollte, übertragen werden kann. Derartige Bewegungsübertragungsmittel sind dagegen relativ leicht derart auszubilden, dass sie bestrahlungsphantomvorbildkonforme Bestrahlungseigenschaften aufweisen. Zum Teil sind derartige Komponenten sogar kommerziell erhältlich.

Insbesondere ist es von Vorteil, wenn bei der Bestrahlungsphantomvorrichtung zumindest eine Stangeneinrichtung und/oder zumindest eine Ketteneinrichtung und/oder zumindest eine Bandeinrichtung und/oder zumindest eine Seileinrichtung und/oder zumindest eine Schneckenantriebseinrichtung und/oder zumindest eine Zahnradantriebseinrichtung vorgesehen ist und/oder bei der Bestrahlungsphantomvorrichtung zumindest ein bestrahlungsphantomvorbildkonformes Kunststoffmaterial verwendet wird. Derartige Kraftübertragungsmittel bzw. derartige Kunststoffmaterialien haben sich in ersten Versuchen als besonders vorteilhaft ergeben. Insbesondere sind damit die erforderlichen Bewegungen besonders vorteilhaft zu übertragen bzw. können unter Verwendung geeigneter Kunststoffmaterialien besonders bestrahlungsphantomvorbildkonforme Eigenschaften realisiert werden. Insbesondere ist es möglich, die genannten Kraftübertragungsmittel zumindest teilweise aus bestrahlungsphantomvorbildkonformem Kunststoffmaterial auszubilden.

Weiterhin ist es von Vorteil, wenn bei der Bestrahlungsphantomvorrichtung zumindest eine Bewegungserzeugungsvorrichtung vorgesehen ist. Bei der Bewegungserzeugungsvorrichtung kann es sich insbesondere um zumindest eine Elektromotoreinrichtung, um zumindest einen Steppermotor, um zumindest einen Linearmotor, um zumindest eine Hydraulikeinrichtung, um zumindest eine Pneumatikeinrichtung und/oder um zumindest eine Roboterarmeinrichtung handeln. Unter Verwendung einer Bewegungserzeugungsvorrichtung können die zur Bewegung der Bestrahlungsphantomvorrichtung erforderlichen Bewegungen vorteilhaft "vor Ort" bzw. in unmittelbarer Nähe hierzu erzeugt werden. Insbesondere die genannten Bewegungserzeugungsvorrichtungen haben sich in ersten Versuchen als besonders vorteilhaft erwiesen. Ganz allgemein hat es sich als vorteilhaft ergeben, wenn die Bewegungserzeugungsvorrichtungen bei Ansteuerung mit einem entsprechenden Steuersignal im Wesentlichen beliebige Richtungen und/oder mit im Wesentlichen beliebigen Geschwindigkeiten (insbesondere solchen, die realistischerweise vom Vorbild erreicht werden können; gegebenenfalls zuzüglich eines Sicherheitszuschlags) bewegt werden können. Auf diese Weise ist ein besonders flexibler Einsatz der Bestrahlungsphantomvorrichtung möglich. Insbesondere können dann auch "zueinander überlagerte" Bewegungen mit lediglich einer bzw. einer reduzierten Anzahl an Bewegungsvorrichtungen erzeugt werden. Bei der Roboterarmeinrichtung kann es sich um einen handelsüblichen Industrieroboter (bzw. einen Teil hiervon) handeln. Bevorzugt ist es übrigens auch, wenn zumindest eine Bewegungserzeugungsvorrichtung einen Messsensor (beispielsweise einen Absolutencoder oder einen Relativ-Messsensor) aufweist. Selbstverständlich ist es möglich, auch eine Mehrzahl von Bewegungsvorrichtungen vorzusehen, beispielsweise um "zueinander überlagerte" Bewegungen zu erzeugen.

Weiterhin ist es von Vorteil wenn bei der Bestrahlungsphantomvorrichtung zumindest eine Bewegungseinrichtung als Schnell-Bewegungseinrichtung und/oder als aperiodische Bewegungsvorrichtung ausgebildet ist. Mit derartigen Bewegungsvorrichtungen können insbesondere auch irreguläre Bewegungsmuster wie beispielsweise Husten und dergleichen simuliert werden. Dementsprechend kann die resultierende Bestrahlungsphantomvorrichtung besonders universell eingesetzt werden, was von Vorteil ist.

Weiterhin ist es vorteilhaft, wenn die Bestrahlungsphantomvorrichtung zumindest zeitweise und/oder zumindest bereichsweise einem realen, insbesondere einem realen biologischen Vorbild nachgebildet ist und besonders bevorzugt zumindest teilweise und/oder zumindest bereichsweise Implantateinrichtungen aufweist. Auf diese Weise lässt sich die Bestrahlungsphantomvorrichtung besonders universell einsetzen bzw. lassen sich besonders aussagekräftige Bestrahlungsvorgangsvalidierungen realisieren. Rein beispielhaft könnte es sich also um eine Art Brustraumnachbildung mit Rippen-Ersatzmaterial, Gewebe-Ersatzmaterial und dergleichen handeln, wenn die Bestrahlung eines Tumors im Brustkorbbereich (auch beispielsweise in der Lunge) validiert werden soll. Selbstverständlich ist es möglich, bei der Bestrahlungsphantomvorrichtung zumindest teilweise auf "Naturmaterialien" zurückzugreifen, wie beispielsweise um echtes Knochenmaterial, insbesondere eines menschlichen Skeletts. Typische radiologische Absorptionseigenschaften sind für Knochen 1,25-2, für Fett 0,8-1 (insbesondere 0,9), für die Lunge 0-0,7 und für Luft 0 (jeweils relativ zur radiologischen Absorptionseigenschaft von Wasser). Eine möglichst detailgetreue Nachbildung eines Vorbilds kann dabei auch Implantate und dergleichen vorsehen. Beispielsweise könnten im Brustkorbbereich Implantate (beispielsweise aus Titanlegierungen) im Bereich der Wirbelsäule vorhanden sein, um diese zu versteifen. Auch andere Implantateinrichtungen sind selbstverständlich denkbar. Die Nachbildung eines realen Vorbilds muss sich dabei nicht notwendigerweise nur auf Bestrahlungseigenschaften des Vorbilds, sondern kann sich insbesondere auch auf andere Effekte beziehen. Beispielsweise kann sich dies auch auf "Angriffspunkte" eines Bewegungssubstitutsmessmittels beziehen. Hier ist beispielsweise an Dehnungsmessstreifen, die um den Brustkorb eines Patienten gelegt werden, zu denken. Auch ist an eine optisches Verfolgungssystem mit einem Kamerasystem (beispielsweise mit einer bzw. einer Mehrzahl an Videokameras), ein implantiertes Tracermaterial im "Tumorbereich", an Lasertriangulationssensoren und sonstige im Stand der Technik bekannte Bewegungssubstitutmessmittel zu denken. Insbesondere ist auch an sogenannte "ground truth"-Verfahren zu denken. Bei diesen wird ein Messwert, von dem bekannt ist, dass er vergleichsweise genaue Werte liefert (vorliegend beispielsweise die Positionsmesswerte des Roboterarms oder aber auch implantierte Goldsensoren, die der Bewegungserfassung dienen) zur Kalibrierung der sonstigen Bewegungssubstitutmesssignalen verwendet. Durch eine derartige Kalibrierung können typischerweise mithilfe der Bewegungssubstitute besonders genaue Messwerte gewonnen werden, auch unter ungünstigen Rahmenbedingungen.

Vorteilhaft ist es weiterhin, wenn bei der Bestrahlungsphantomvorrichtung zumindest eine elektronische Steuereinrichtung vorgesehen ist, welche bevorzugt zumindest eine Datenspeichereinrichtung und/oder zumindest eine Datenaufnahmeeinrichtung aufweist. Mit Hilfe der zumindest einen Datenspeichereinrichtung und/oder der zumindest einen Datenaufnahmeeinrichtung (welche insbesondere als Messsensor oder dergleichen Messmittel ausgebildet sein kann) ist es möglich, die bei der Bestrahlungsphantomvorrichtung gewonnenen Erkenntnisse insbesondere einer quantitativen Auswertung zuzuführen. Beispielsweise können die Bewegungen von einzelnen Bestrahlungsphantomvorrichtungsbereichen zeit- und ortsaufgelöst gespeichert werden. Im Übrigen ist hier nicht nur an digitale Datenspeichereinrichtungen und/oder Datenaufnahmeeinrichtungen zu denken, sondern auch an analoge Einrichtungen (wie beispielsweise röntgenempfindliche Filme und dergleichen). Bevorzugt kann die Datenaufnahme und/oder die Datenspeicherung auch zeitaufgelöst erfolgen, wodurch in der Regel eine besonders hochwertige Verifikation des Bestrahlungsvorgangs möglich ist. Selbstverständlich ist es auch möglich, dass Messwerte der Bestrahlungsvorrichtung (oder sonstiger Vorrichtungen) mitprotokolliert werden. Beispielsweise können die Bewegungen der einzelnen Bestrahlungsphantomvorrichtungsbereiche zeitlich synchronisiert zum Bestrahlungsvorgang gemessen, gespeichert und anschließend ausgewertet werden. Eine Aufnahme einer größeren Anzahl von Messsignalen (die gegebenenfalls auch über eine entsprechende Bandbreite verfügen) setzt selbstverständlich eine geeignete ausgebildete Datenspeichereinrichtung bzw. Datenaufnahmeeinrichtung voraus. Insbesondere müssen entsprechend viele "Datenspuren" vorhanden sein (beispielsweise durch entsprechend viele Messkanäle), die auch jeweils eine entsprechende Menge an Daten verarbeiten können müssen. Beispielsweise könnte sich bei einem der Messkanäle um eine Videokamera handeln, deren Bilddaten im MP4-Format gespeichert werden. Alternativ wird in verschiedenen Datenspuren die Zeit und die je 6 Komponenten einer 6D Bewegung (Translation und Rotation) pro Bewegung pro Bereich gespeichert. Dementsprechend breitbandig muss der entsprechende Datenkanal ausgelegt sein. Die zumindest eine Datenspeichereinrichtung und/oder die zumindest eine Datenaufnahmeeinrichtung kann jedoch zusätzlich oder alternativ auch zur Ansteuerung der Bestrahlungsphantomvorrichtung genutzt werden. Beispielsweise ist es möglich, dass bestimmte Bewegungsabläufe (einschließlich irregulärer Bewegungsabläufe wie Husten und dergleichen) in einer Datenspeichereinrichtung gespeichert sind und während der Validierung der Bestrahlung "abgerufen" werden können. Auch Datenaufnahmeeinrichtungen können sich in diesem Zusammenhang als sinnvoll erweisen, beispielsweise indem ein realer (gegebenenfalls in Folge zu therapierender Patient, oder einfach ein Experimentator "vermessen" wird, und dessen Bewegungen von der Bestrahlungsphantomvorrichtung nachgebildet werden. Dies kann im Übrigen auch Bewegungssubstitute umfassen.

Weiterhin kann die Bestrahlungsphantomvorrichtung zumindest eine Detektoreinrichtung aufweisen. Bei der Detektoreinrichtung kann es sich insbesondere um zumindest eine Filmdetektoreinrichtung und/oder um zumindest eine zeitaufgelöste Detektoreinrichtung handeln. Besonders vorzugsweise kann die Detektoreinrichtung (insbesondere auch die Filmdetektoreinrichtung) auswechselbar angeordnet sein. Wie bereits erwähnt, lassen sich bei Verwendung von Detektoreinrichtungen besonders genaue, insbesondere auch quantitative Validierungen einer Bestrahlung realisieren. Obgleich Halbleiterdetektoren und sonstige elektronische Detektoren eine gewisse Bedeutung zur Datenaufnahme gewonnen haben, sind nach wie vor Filmdetektoren ein Standardmittel zur Strahlungsmessung. Auch einige Definitionen bzw. Vorschriften beruhen auf der Verwendung von Filmdetektoreinrichtungen. Zusätzlich oder alternativ können auch zeitaufgelöste Detektoreinrichtungen, wie beispielsweise Halbleiterdetektoren, bevorzugt sogenannte "Pin Point"-lonisationskammerdetektoren vorgesehen werden. Diese weisen zwar eine im Vergleich zu Filmdetektoren in der Regel deutlich schlechtere Ortsauflösung auf, die Möglichkeit einer zeitaufgelösten Messung bietet jedoch üblicherweise nicht zu unterschätzende Vorteile. Besonders vorteilhaft ist es, wenn in einem Detektorkopf beide Messprinzipien (also zeitaufgelöste Detektoreinrichtungen und Filmdektoreinrichtungen) miteinander kombiniert sind. In diesem Fall können die Vorteile "aus beiden Welten" genutzt werden. Eine besonders rasche Auswechselbarkeit der Detektoreinrichtung ermöglicht einen schnellen Wechsel im Schadensfall bzw. zwischen zwei Bestrahlungsvalidierungen (gegebenenfalls auch Teil-Bestrahlungsvorgangsvalidierungen). Hierzu können an sich bekannte Schnellverschlüsse und dergleichen genutzt werden. Gerade bei der Verwendung (in der Regel elektrisch arbeitender) zeitaufgelöster Detektoreinrichtungen können auch entsprechend vielpolige elektrische Steckverbinder verwendet werden.
Ein weiterer bevorzugter Aufbau der Bestrahlungsphantomvorrichtung ergibt sich, wenn diese zumindest teilweise modular aufgebaut ist, wobei insbesondere Module und/oder Teile von Modulen austauschbar ausgebildet sind.

Auf diese Weise können die entsprechenden Teile schnell und einfach gewechselt werden, so dass eine Anpassung der Bestrahlungsphantomvorrichtung auf unterschiedliche "Vorbilder" schnell, einfach und kostengünstig möglich ist. Dies kann eine besonders universelle Anwendbarkeit der Bestrahlungsphantomvorrichtung nochmals deutlich erhöhen.

Weiterhin wird ein Verfahren zur Validierung des Bestrahlungsvorgangs vorgeschlagen, wobei der Bestrahlungsvorgang an einer Bestrahlungsphantomvorrichtung erfolgt, die zumindest eine Bewegungsvorrichtung zur Bewegung von zumindest einem ersten Teilbereich der Bestrahlungsphantomvorrichtung, insbesondere relativ zu zumindest einem zweiten Teilbereich der Bestrahlungsphantomvorrichtung aufweist, und wobei die Bestrahlungsphantomvorrichtung zumindest zeitweise und/oder zumindest bereichsweise bestrahlungsphantomvorbildkonforme Bestrahlungseigenschaften aufweist. Bei der Durchführung des vorgeschlagenen Verfahrens ergeben sich bereits die vorab genannten Vorteile und Eigenschaften zumindest in analoger Weise. Darüber hinaus ist es möglich, dass Verfahren im Sinne der vorherigen Beschreibung zumindest in Analogie weiterzubilden. Insbesondere ist es möglich eine entsprechend große Anzahl an Messdaten zu gewinnen, so dass gerade auch 4D-Bestrahlungsplanungen validiert und optimiert werden können. Insbesondere auf Basis von (gemessenen) Bewegungsdaten und Bewegungssurrogatdaten können 4D-Dosisverteilungen nachgerechnet werden, und mit den in der Bestrahlungsphantomvorrichtung gemessenen Strahlungseinträgen verglichen werden.

Insbesondere ist es möglich, dass zur Durchführung des Verfahrens zumindest zeitweise und/oder zumindest bereichsweise eine Bestrahlungsphantomvorrichtung mit dem vorab beschriebenen Aufbau verwendet wird. In diesem Falle ergeben sich die bereits beschriebenen Vorteile und Eigenschaften in analoger Weise.

Insbesondere ist es möglich, dass das Verfahren derart durchgeführt wird, dass Störsignale verwendet werden, welche insbesondere in einer Speichereinrichtung abgelegt sind und/oder durch zumindest eine Messvorrichtung gewonnen werden. Bei einer derartigen Weiterbildung des Verfahrens ist es insbesondere möglich "irreguläre" Bewegungen und deren Einfluss auf die Bestrahlung zu verifizieren. Hier kann es sich beispielsweise um ein Husten eines Patienten (bzw. vorliegend der Bestrahlungsphantomvorrichtung) handeln, wenn "ein Tumor im Brustbereich" behandelt werden soll. Mit einem derartigen Verfahren kann mit der Bestrahlungsphantomvorrichtung die Realität besonders exakt nachgebildet werden.

Weiterhin ist es zusätzlich oder alternativ möglich, die Robustheit von sogenannten "gating"-Bestrahlungsverfahren zu validieren. Dies gilt insbesondere, wenn die bereits erwähnten "überlagerten" Bewegungsmuster verwendet werden. So beruht beispielsweise bei "gating"-Bestrahlungsverfahren das "gating" oftmals auf der Atembewegung. Ist die Atembewegung durch eine (zusätzliche) Tumorbewegung überlagert, so kann es bei der Bestrahlung des Tumors zu (unerwünschten) Artefakten kommen. Mit der vorgeschlagenen Bestrahlungsphantomvorrichtung bzw. dem Vorgeschlagenenverfahren können auch hierzu Untersuchungen bzw. Validierungen durchgeführt werden.

Im Folgenden wird die Erfindung anhand vorteilhafter Ausführungsbeispiele und unter Bezugnahme auf die beigefügte Zeichnung näher erläutert. Es zeigen:
- Fig. 1:: ein Ausführungsbeispiel für einen Bestrahlungsphantommessaufbau in einer schematischen Überblicksansicht;
- Fig. 2:: einen Querschnitt durch ein mögliches Ausführungsbeispiel eines Bestrahlungsphantoms;
- Fig. 3:: ein möglicher Aufbau eines Detektorkopfs, jeweils in schematischer Draufsicht aus unterschiedlichen Raumrichtungen;
- Fig. 4:: ein mögliches Verfahren zur Validierung eines Bestrahlungsvorgangs in einem schematischen Flussdiagramm.

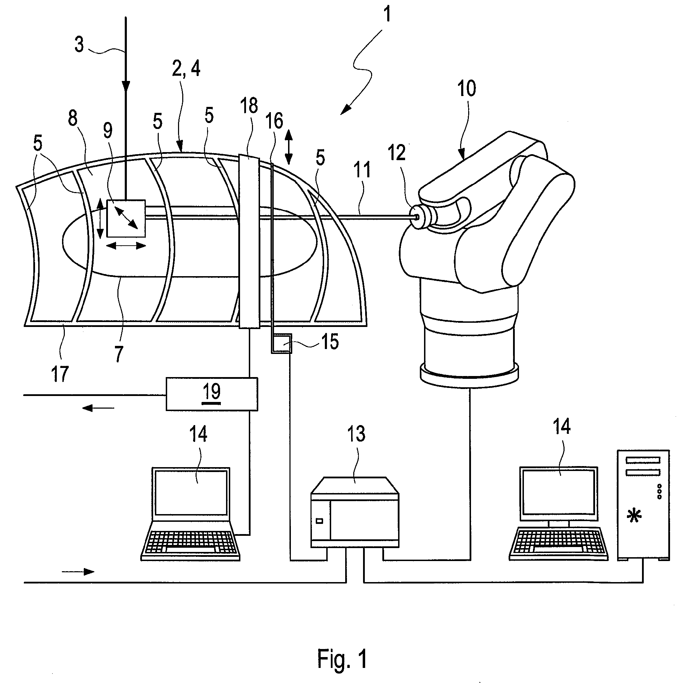

In Fig. 1 ist in einer schematischen Überblicksansicht ein Bestrahlungsphantommessaufbau 1 dargestellt. Der Bestrahlungsphantommessaufbau 1 weist mehrere Komponenten auf, die im Folgenden jeweils einzeln sowie in ihrem Zusammenhang zueinander näher erläutert werden.

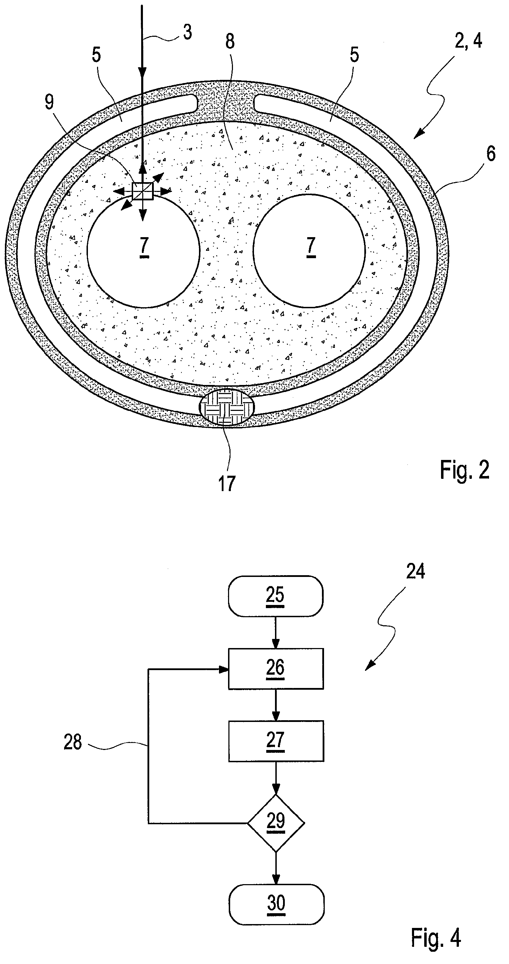

Ein wesentlicher Bestandteil des Bestrahlungsphantommessaufbaus 1 ist das eigentliche Bestrahlungsphantom 2 (siehe hierzu auch Fig. 2). In vorliegendem Fall wird mit dem Bestrahlungsphantom 2 ein Brustkorb 4 nachgebildet, wobei ein Tumor, welcher im Lungengewebe sitzt, durch einen Schwerionenstrahl 3 behandelt werden soll. Der Schwerionenstrahl 3 wird auf an sich bekannte Weise erzeugt (typischerweise mit Hilfe eines Linearbeschleunigers und/oder eines Teilchensynchrotrons) und vorliegend im sogenannten Raster-Scan-Verfahren über den Zielvolumenbereich geführt, der von seinen räumlichen Abmessungen her im Wesentlichen der Tumorausdehnung (zuzüglich eines Sicherheitszuschlags) entspricht. Auch die Berechnungsverfahren zur Erstellung einer sogenannten Bestrahlungsplanung, d. h. die "Umrechnung" der vorgegebenen medizinischen Bedingungen auf einen Parametersatz zur Ansteuerung des Teilchenstrahlbeschleunigers, der den Schwerionenstrahl 3 erzeugt und geeignet ablenkt, ist an sich bekannt und soll im Folgenden nicht weiter beschrieben werden.

Aus Sicherheitsgründen soll vorliegend der berechnete Bestrahlungsplan vorab in ein Bestrahlungsphantom 2 "geschossen" werden, um die Güte bzw. "Korrektheit" vorab zu testen. Dies kann einerseits im realen Behandlungsbetrieb sinnvoll sein, insbesondere dann, wenn ein besonders komplex geformter Tumor vorliegt, der Tumor benachbart zu einem besonders kritischen Gewebebereich liegt und/oder das Applikationsverfahren stark von den Bewegungen des Patienten beeinflusst wird (derart, dass eine dosimetrische Veränderung feststellbar ist). Vorteilhaft ist eine Validierung einer Bestrahlungsplanung an einem Bestrahlungsphantom 2 jedoch insbesondere auch zu Forschungszwecken, beispielsweise um neuartige Berechnungsalgorithmen zur Berechnung einer Bestrahlungsplanung und/oder neuartige Strahlansteuerungssysteme, neuartige Strahlnachführungsysteme usw. zu entwickeln und zu überprüfen.

Im vorliegend Ausführungsbeispiel handelt es sich bei dem Bestrahlungsphantom 2 um einen "künstlichen Brustkorb" 4. Ähnlich zu einem "echten Brustkorb" weist der vorliegende Brustkorb 4 eine anatomisch korrekte Anzahl an einzelnen Rippen 5 auf. Im vorliegenden Fall sind die Rippen aus einem Kunststoffmaterial (vorliegend PVC) gefertigt. Es ist jedoch ebenso denkbar, dass als Basis für den Brustkorb 4 ein echtes menschliches Skelett (eines Verstorbenen) verwendet wird. Die einzelnen Rippen 5 sind in einer dünnen Kautschukschicht 6 eingebettet. Die Kautschukschicht 6 ist im Wesentlichen geschlossen ausgebildet (abgesehen vom "oberen" und "unteren" Ende des Brustkorbs 4) und simuliert die Strahlungseigenschaften (gegebenenfalls einschließlich der Reflektionseigenschaften, die beispielsweise bei der Verwendung eines Stereokamerasystems als Bewegungsurrogat von Bedeutung sind) von Haut sowie von Muskelgewebe. Der Brustkorb 4 ist auf einer Grundplatte aus Epoxydharz gelagert.

Zur Simulation der Lungenflügel sind zwei luftgefüllte Ballons 7 vorgesehen, die gegebenenfalls über eine Balganordnung periodisch unterschiedlich stark mit Luft gefüllt werden können, und dadurch vergrößert bzw. verkleinert werden können. Die luftgefüllten Ballons 7 sind besonders gut in Fig. 2 zu erkennen, die einen schematischen Querschnitt durch den Brustkorb 4 des Bestrahlungsphantoms 2 zeigt. Die luftgefüllten Ballons 7 können vorzugsweise mit einem luftdurchlässigen Schaummaterial geringer Dichte befüllt werden, um dem Vorbild von echtem Lungengewebe (insbesondere hinsichtlich der Strahlungseigenschaften) möglichst nahe zu kommen. Das sonstige innere Gewebe des Brustkorbs 4 wird vorliegend durch einen elastischen Polyurethan-Schaum 8 simuliert, mit dem der Innenraum des Brustkorbs 4 (abgesehen von den luftgefüllten Ballons 7) weitgehend gefüllt ist.

Vorzugsweise kann der Brustkorb 4 durch geeignete vorhandene Schnittstellen geöffnet werden, so dass die hierin befindlichen "Weichteile" schnell und einfach gewechselt werden können. Dies betrifft insbesondere den Polyurethan-Schaum 8 sowie den Detektorkopf 9 (einschließlich dazugehöriger "Hilfsmittel", wie beispielsweise der Kunststoffstange 11). Gegebenenfalls ist es auch möglich, dass die "Weichteile" über das "obere" bzw. das "untere" Ende des Brustkorbes 4 herausgezogen bzw. in diesen hinein gesteckt werden können.

Selbstverständlich können anstelle der genannten Materialien andere geeignete Materialien verwendet werden. Insbesondere sollten diese hinsichtlich ihrer Eigenschaften gegenüber Strahlung (insbesondere Schwerionenstrahlung 3 bzw. Röntgenstrahlung eines CTs) dem "Vorbild" besonders nahe kommen.

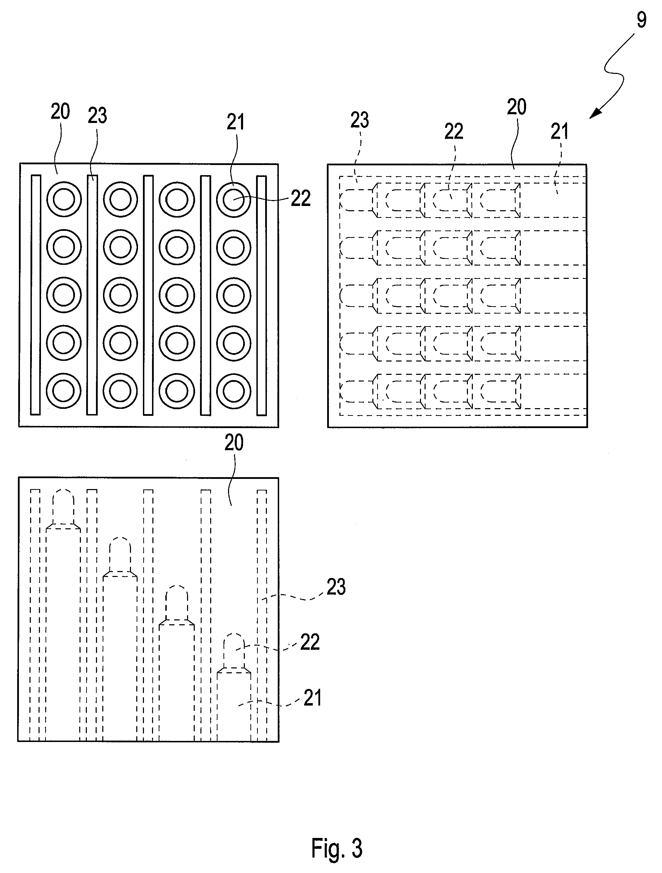

Da im vorliegend dargestellten Ausführungsbeispiel die Bestrahlungsplanung für einen an einem Lungenflügel sitzenden Tumor verifiziert werden soll, ist quasi als "Ersatz" für den Tumor (bzw. für den Zielvolumenbereich sowie das hierzu angrenzende Volumen) ein Detektorkopf 9 angeordnet, der in sämtlichen drei Raumrichtungen translatorisch bewegt werden kann (in Fig. 1 und 2 jeweils durch Doppelpfeile angedeutet). Darüber hinaus kann der Detektorkopf 9 auch rotatorisch bewegt werden, was vorliegend aus Veranschaulichungsgründen nicht näher dargestellt wird. Ein möglicher Aufbau eines Detektorkopfs 9 ist in Fig. 3 näher dargestellt. Vorzugsweise ist der Detektorkopf 9 im Wesentlichen vollständig und "spaltfrei" in den ihn umgebenden elastischen Polyurethan-Schaum 8 eingebettet. Bei einer Bewegung des Detektorkopfs 9 bewegt sich somit (zumindest) der benachbarte Bereich des Polyurethan-Schaums 8 mit (einschließlich hierdurch hervorgerufene Verformungen, Stauchungen und Dehnungen).

Der Detektorkopf 9 kann beim vorliegend dargestellten Ausführungsbeispiel von einem kommerziell erhältlichen Industrieroboter 10 bewegt werden. Da der Industrieroboter 10 zu einem großen Teil aus Metall besteht, muss dieser in einer gewissen Entfernung vom Zielvolumenbereich bzw. vom Detektorkopf 9 entfernt stehen, damit das Messergebnis nicht unrealistisch verfälscht wird. Hierfür ist beim vorliegend dargestellten Ausführungsbeispiel eine Kunststoffstange 11 vorgesehen, die die Werkzeugaufnahme 12 des Industrieroboters 10 mit dem Detektorkopf 9 verbindet. Zusätzlich können in der Kunststoffstange 11 vorliegend nicht dargestellte dünne Messleitungen zur Weiterleitung von Messsignalen, die im Detektorkopf 9 gewonnen werden, aufgenommen sein. Die Kunststoffstange 11 ist aus einem geeigneten Kunststoffmaterial gefertigt, sodass diese bezüglich ihrer Eigenschaften gegenüber Strahlung (insbesondere Teilchenstrahlung und/oder Röntgenstrahlung) einem realen Brustkorb möglichst nahe kommt. Dabei kann es durchaus sinnvoll sein, dass die Kunststoffstange 11 mehrteilig aufgebaut ist, wobei unterschiedliche Kunststoffmaterialen zur Anwendung kommen können. Wie man Fig. 1 entnehmen kann, verläuft die Kunststoffstange 11 zu einem großen Teil durch den luftgefüllten Hohlraum hindurch, der von den luftgefüllten Ballons 7 (welche gegebenenfalls mit einem luftdurchlässigen Schaummaterial gefüllt sind) gebildet wird. Gegebenenfalls kann das Schaummaterial eine geeignet dimensionierte Aussparung für die Kunststoffstange 11 aufweisen. Dadurch erfolgt bei einer Bewegung der Kunststoffstange 11 eine möglichst geringe Verformung des benachbart zur Kunststoffstange 11 liegenden Polyurethan-Schaums 8 in sich, welcher Effekte nach sich ziehen kann, die vom "Original" eines Patienten abweichen können (denn ein Mensch hat keine derartige Struktur).

Auch wenn dies vorliegend nicht näher dargestellt ist, ist es sinnvoll, im Bestrahlungphantom 2 weitere Detektorköpfe (gegebenenfalls auch einen einzelnen zusätzlichen Detektorkopf) vorzusehen. Insbesondere können Detektorköpfe im Bereich von (einem oder mehreren) Risikoorganen und/oder im Bereich von Normalgewebe vorgesehen werden, um hier die entsprechende eingetragene Strahlungsdosis zu messen. Je nach Erfordernis können die entsprechenden Detektorköpfe bewegt und/oder unbewegt ausgeführt sein.

Die Ansteuerung des Industrieroboters 10 erfolgt durch einen kommerziell erhältlichen Controller 13. Wie üblich, kann der Bestrahlungsphantommessaufbau 1 durch einen oder mehrere Benutzerterminals 14 gesteuert und überwacht werden.

Weiterhin ist in Fig. 1 ein Schrittmotor 15 zu erkennen, der mit einem flexiblen Band 16 verbunden ist, das um den Brustkorb 4 herum gelegt ist. Die Anordnung ist so gewählt, dass die Länge des flexiblen Bands 16 vom Schrittmotor 15 geändert werden kann, sodass auf diese Weise (im Zusammenspiel mit dem elastischen Bauschaum 8) der Brustkorb 4 gesteuert gehoben und gesenkt werden kann, wodurch eine Atembewegung simuliert werden kann. Auch der Schrittmotor 15 wird durch den Controller 13 geeignet angesteuert. Selbstverständlich kann auch eine Mehrzahl an Schrittmotoren 15 und/oder an flexiblen Bändern 16 vorgesehen werden, die insbesondere einen gewissen seitlichen Versatz zueinander aufweisen können. Eine anderweitige Ausbildungsweise wäre, dass der Schrittmotor 15 einen Seilzug ansteuert, der (in Kombination mit den elastischen Eigenschaften des Brustkorbes 4) ein "physiologisches" Heben und Senken des Brustkorbs 4 bewirkt.

Die Ansteuerung von Schrittmotor 15 und Industrieroboter 10 kann insbesondere derart erfolgen, dass eine bei realen Patienten oftmals zu beobachtende "einander überlagernde" Bewegung des Tumorbereichs simuliert wird.

Das "Heben" und "Senken" des Brustkorbs 4 hat nicht nur dahingehend einen Effekt, dass sich die Rippen 5 im Verhältnis zur Wirbelsäule 17 bewegen und sich das "Gewebe" im Inneren des Brustkorbs 4 (also insbesondere der elastische Bauschaum 8) verformen (insbesondere gestaucht bzw. gedehnt werden) kann. Vielmehr wird das Heben und Senken eines Brustkorbs in der Realität oftmals zur Verwendung eines sogenannten Bewegungssubstitut-Signals genutzt. Hierzu sind unterschiedliche Verfahren bekannt. Ein mögliches Verfahren ist eine Messaufnahme mit Hilfe eines Dehnungsmessstreifens 18, der um den Brustkorb 4 gelegt wird (sogenannter ANZAI-Streifen). Dieses Verfahren wird auch vorliegend beim Bestrahlungsphantommessaufbau 1 verwendet. Die so gewonnenen Daten werden von einem Messaufnehmer 19 aufgenommen und geeignet verarbeitet. Die Ausgangssignale des Messaufnehmers 19 werden beispielsweise dazu genutzt, den Schwerionenstrahl 3 nachzusteuern (beispielsweise bei sogenannten Tracking-Verfahren) oder in Extremfällen auch kurzzeitig auszuschalten. Lediglich der Vollständigkeit halber sollte darauf hingewiesen werden, dass bei so genannten "gating"-Verfahren ein kurzfristiges Ausschalten des Schwerionenstrahls 3 standardmäßig erfolgt.

Wie man Fig. 1 entnehmen kann, wird es durch die Verwendung eines (hinreichend schnellen) Schrittmotors 15 sowie eines (hinreichend schnellen) Industrieroboters 10 möglich, einerseits im Wesentlichen beliebige Bewegungsabläufe zu simulieren, und darüber hinaus auch sehr schnelle Bewegungsabläufe zu simulieren. Somit ist man also nicht auf ein stets wiederkehrendes Bewegungsmuster beschränkt, sondern es können auch beispielsweise Änderungen im Atemverhalten des Patienten simuliert werden. Insbesondere durch die schnelle Veränderbarkeit von Schrittmotor 15 und Industrieroboter 10 können auch schnelle Bewegungen wie Schluckauf, Husten und dergleichen simuliert werden. Weiterhin ist es möglich, dass Schrittmotor 15 und Industrieroboter 10 aufeinander synchronisiert sind und es dadurch möglich wird, sich zeitlich verändernde Verläufe (zum Beispiel Phasenverschiebung zwischen den Bewegungen von "Brustkorb" und "Tumor") zu untersuchen. Insbesondere können solche Effekte eingehender untersucht werden, die einen größeren dosimetrischen Einfluss haben können. Insbesondere ist es dadurch möglich, die Robustheit einer Bestrahlungsplanung bzw. eines Bestrahlungsverfahrens gegenüber derartigen Effekten zu überprüfen.

Der Detektorkopf 9 ist insbesondere in Fig. 3 eingehender dargestellt. Der Detektorkopf 9 ist dabei über eine Schnellverbindung mit der Kunststoffstange 11 (und damit mit dem Industrieroboter 10) verbunden. Auf diese Weise ist es möglich, den gesamten Detektorkopf 9 schnell austauschen zu können, beispielsweise um neue Röntgenfilme (im Weiteren näher erläutert) einzulegen, ohne dass der Bestrahlungsphantommessaufbau 1 über einen längeren Zeitraum hinweg "blockiert" ist. Selbst dann, wenn nicht der gesamte Detektorkopf 9 ausgetauscht wird, ermöglicht es der vorliegend gewählte Aufbau des Detektorkopfs 9 mit schlitzartigen Ausnehmungen 23, die Röntgenfilme schnell zu wechseln, so dass auch hierdurch Standzeiten verkürzt werden können.

Der Detektorkopf 9 ist eine Variation des in der Veröffentlichung "A system for three-dimensional dosimetric verification of treatment plans in intensitymodulated radiotherapy with heavy ions" von C. Karger, O. Jäkel, G. Hartmann und P. Heeg in Med. Phys. 26 (10) vom Oktober 1999, Seite 2.125 bis 2.132 beschriebenen Detektorkopfs. Der im Wesentlichen würfelförmige Basiskörper 20 des Detektorkopfs 9 besteht aus einem Plexiglasmaterial. Im Basiskörper 20 sind in einer Array-Anordnung (4 x 5 Array) insgesamt 24 abgestufte Senklöcher 21 mit unterschiedlichen Tiefen angebracht. Die Lage und Ausbildung der Senklöcher 21 ist dabei durch die insgesamt drei Draufsichten aus unterschiedlichen Raumrichtungen, die in Fig. 3 dargestellt sind, gut zu erkennen. Die abgestuften Senklöcher 21 dienen der Aufnahme von stiftförmigen Ionisationskammern (sogenannte "pin point"-lonisationskammern). Der jeweilige Vorderteil 22 der Senklöcher 21 beherbergt dabei das eigentliche empfindliche Messvolumen, das typischerweise ein Volumen von 2 - 3 mm³ aufweist. Derartige stiftförmige lonisationskammern weisen eine gute Messgenauigkeit bezüglich der eingestrahlten Dosis auf, und sind darüber hinaus auch zeitauflösend. Problematisch bei diesen ist jedoch die in aller Regel vergleichsweise geringe Ortsauflösung.

Um die Ortsauflösung der Bestrahlungsvalidierung zu erhöhen sind daher beim vorliegend verwendeten Detektorkopf 9 zusätzliche schlitzartige Ausnehmungen 23 vorgesehen, die jeweils zwischen zwei Reihen zueinander benachbarter abgestufter Senklöcher 21 ausgebildet sind. Die schlitzartigen Ausnehmungen 23 dienen der Aufnahme von Röntgenfilmen. Röntgenfilme weisen eine sehr hohe Ortsauflösung auf (wenn auch eine vergleichsweise niedrige Dosisauflösung und darüber hinaus keine Zeitauflösung). Sinnvoll kann es im Übrigen sein, wenn sich die Röntgenfilme in lichtdichten Schutztaschen befinden. In diesem Fall können die Röntgenfilme bei Tageslicht gehandhabt werden, was deren Verwendung deutlich vereinfacht. Insbesondere kann ein Austausch der Röntgenfilme im Detektorkopf 9 erfolgen, ohne dass zunächst eine Dunkelkammer aufgesucht werden muss.

Durch die Kombination von stiftförmigen lonisationskammern und Röntgenfilmen kann die Gesamtgenauigkeit des Detektorkopfs 9 deutlich erhöht werden. Er eignet sich daher im besonderen Maße für das vorgeschlagene Validierungsverfahren.

Im Übrigen können im oder am Detektorkopf 9 auch (beispielsweise radioopaque) Marker oder Transponder angebracht werden, die eine Lokalisierung (Positionsbestimmung) des Detektors, beispielsweise durch Röntgen oder sonstige externe Überwachungssysteme ermöglichen. Eine derartige Positionsbestimmung wird zum Teil auch bei "realen Patienten" durchgeführt, so dass hierdurch ein besonders genaues Abbild der Realität erfolgen kann.

In Fig. 4 ist schließlich noch ein Verfahren 24 zur Validierung eines Bestrahlungsvorgangs in einem schematischen Flussdiagramm dargestellt.
In einem ersten Schritt 25 wird zunächst die vorgesehene (errechnete) Bestrahlungsplanung eingelesen, die anschließend auf das Bestrahlungsphantom 2 appliziert werden soll. Anschließend wird in einem zweiten Schritt 26 die eigentliche Bestrahlung durchgeführt. Dabei wird nicht nur der Schwerionenstrahl 3 geeignet moduliert (insbesondere auch in Abhängigkeit der vom Dehnungsmessstreifen 18 oder sonstiger Messgeräte gelieferten Messdaten), sondern gleichzeitig auch das Bestrahlungsphantom 2 geeignet bewegt. Geeignete Bewegungen können dabei insbesondere auch irreguläre Bewegungen wie beispielsweise Husten und dergleichen umfassen. Insbesondere können auch veränderte Korrelationen zwischen Brustkorb 4 und Tumorbewegung durchgeführt werden.

Während der Bestrahlung werden die im Detektorkopf 9 gemessenen Daten, die der eigentlichen Bestrahlungsvalidierung dienen, zeitlich synchronisiert aufgenommen und gespeichert (Schritt 27). Beispielsweise können hierbei die Bewegungen der Detektorköpfe 9, der Verlauf des Bewegungssurrogats (vorliegend ein Dehnungsmessstreifen) 18 und weitere Daten der Strahlapplikation (insbesondere der Bestrahlungszeitpunkt jedes Rasterpunkts) aufgenommen und gespeichert werden. Die Schleife aus Bestrahlungsdurchführung 26 und Messwertaufnahme 27 wird so oft durchlaufen (Rücksprung 28), bis die gesamte Bestrahlungsplanung appliziert wurde. Dies wird in einem Überprüfungsschritt 29 überprüft.

Wird im Überprüfungsschritt 29 festgestellt, dass die gesamte Bestrahlungsplanung appliziert wurde, so endet 30 das Verfahren.

**Bezugszeichenliste:**

| | | | |
|---|---|---|---|
| 1. | Bestrahlungsphantommessaufbau | 16. | Flexibles Band |
| | | 17. | Wirbelsäule |
| 2. | Bestrahlungsphantom | 18. | Dehnungsmessstreifen |
| 3. | Schwerionenstrahl | 19. | Messaufnehmer |
| 4. | Brustkorb | 20. | Basiskörper |
| 5. | Rippen | 21. | Senklöcher |
| 6. | Kautschukschicht | 22. | Vorderteil |
| 7. | Luftgefüllte Ballons | 23. | Schlitzartige Ausnehmung |
| 8. | ,Polyurethan-Schaum | 24. | Verfahren |
| 9. | Detektorkopf | 25. | Einlesen der Bestrahlungsplanung |
| 10. | Industrieroboter | | |
| 11. | Kunststoffstange | 26. | Bestrahlungsdurchführung |
| 12. | Werkzeugaufnahme | 27. | Datenaufnahme |
| 13. | Controller | 28. | Rücksprung |
| 14. | Benutzerterminal | 29. | Überprüfungsschritt |
| 15. | Schrittmotor | 30. | Ende |

## Patentansprüche

1. Bestrahlungsphantomvorrichtung (1) insbesondere Bestrahlungsphantomvorrichtung (1) zur Validierung eines Bestrahlungsvorgangs (26), bevorzugt zur Validierung einer Bestrahlungsplanung, aufweisend
ein Bestrahlungsphantom (2) und
zumindest eine Bewegungsvorrichtung (10, 15) zur Bewegung von zumindest einem ersten Teilbereich (5, 6, 7, 8, 9) des Bestrahlungsphantoms (2) relativ zu zumindest einem zweiten Teilbereich (5, 6, 7, 8, 9, 17) des Bestrahlungsphantoms (2), wobei das Bestrahlungsphantom (2) zumindest zeitweise und/oder zumindest bereichsweise bestrahlungsphantomvorbildkonforme Bestrahlungseigenschaften aufweist, **dadurch gekennzeichnet, dass** eine Bewegungsvorrichtung (10) derart ausgebildet ist, dass sie eine überlagerte Bewegung zweier Einzel-Bewegungsmuster erzeugen kann sowie zumindest zeitweise und/oder zumindest bereichsweise zumindest eine zeitabhängige 6D-Bewegung durchführen kann.

2. Bestrahlungsphantomvorrichtung (1) nach Anspruch 1, **dadurch gekennzeichnet, dass** das Bestrahlungsphantom (2) zumindest bereichsweise bestrahlungsphantomvorbildkonforme Behandlungsstrahleigenschaften (3) und/oder zumindest bereichsweise bestrahlungsphantomvorbildkonforme Messstrahleigenschaften aufweist.

3. Bestrahlungsphantomvorrichtung (1) nach Anspruch 1 oder 2, **dadurch gekennzeichnet dass** die das Bestrahlungsphantom (2) (1) zumindest bereichsweise bestrahlungsphantomvorbildkonforme Abschwächungseigenschaften und/oder bestrahlungsphantomvorbildkonforme Sekundärstrahlungsemissionseigenschaften und/oder bestrahlungsphantomvorbildkonforme Streuungseigenschaften aufweist, insbesondere für benachbart und/oder proximal und/oder scheibenartig-axial und/oder kegelartig zu einem Zielvolumenbereich (9) liegende Teilbereiche (5, 6, 7, 8) des Bestrahlungsphantoms (2).

4. Bestrahlungsphantomvorrichtung (1) nach einem der vorangehenden Ansprüche, **dadurch gekennzeichnet dass** das Bestrahlungsphantom (2) zumindest bereichsweise bestrahlungsphantomvorbildkonforme Reflektionseigenschaften und/oder bestrahlungsphantomvorbildkonforme Sekundärstrahlungsemissionseigenschaften und/oder bestrahlungsphantomvorbildkonforme Streuungseigenschaften aufweist, insbesondere für benachbart und/oder distal und/oder scheibenartig-axial und/oder kegelartig zu einem Zielvolumenbereich (9) liegende Teilbereiche (5, 6, 7, 8, 17) des Bestrahlungsphantom (2).

5. Bestrahlungsphantomvorrichtung (1) nach einem der vorangehenden Ansprüche, **gekennzeichnet durch** zumindest ein Bewegungsübertragungsmittel (11, 16), welches zumindest bereichsweise bestrahlungsphantomvorbildkonforme Bestrahlungseigenschaften aufweist.

6. Bestrahlungsphantomvorrichtung (1) nach einem der vorangehenden Ansprüche, **gekennzeichnet durch** zumindest eine Bewegungserzeugungsvorrichtung (10, 15), insbesondere zumindest eine Elektromotoreinrichtung (15), zumindest einen Steppermotor (15), zumindest einen Linearmotor, zumindest eine Hydraulikeinrichtung, zumindest eine Pneumatikeinrichtung und/oder zumindest eine Roboterarmeinrichtung (10).

7. Bestrahlungsphantomvorrichtung (1) nach einem der vorangehenden Ansprüche, **dadurch gekennzeichnet, dass** das Bestrahlungsphantom (2) zumindest bereichsweise einem realen, insbesondere biologischen Vorbild nachgebildet ist und besonders bevorzugt zumindest teilweise und/oder zumindest bereichsweise Implantateinrichtungen aufweist.

8. Bestrahlungsphantomvorrichtung (1) nach einem der vorangehenden Ansprüche, **gekennzeichnet durch** zumindest eine Steuereinrichtung (13, 14, 19), insbesondere **durch** zumindest eine elektronische Steuereinrichtung (13, 14, 19), welche bevorzugt zumindest eine Datenspeichereinrichtung und/oder zumindest eine Datenaufnahmeeinrichtung (19) aufweist.

9. Bestrahlungsphantomvorrichtung (1) nach einem der vorangehenden Ansprüche, **gekennzeichnet durch** zumindest eine Detektoreinrichtung (9, 21, 23), insbesondere **durch** zumindest eine Filmdetektoreinrichtung (9, 21, 23) und/oder **durch** und/oder um zumindest eine zeitaufgelöste Detektoreinrichtung, welche vorzugsweise auswechselbar angeordnet ist.

10. Bestrahlungsphantomvorrichtung (1) nach einem der vorangehenden Ansprüche, **dadurch gekennzeichnet, dass** diese zumindest teilweise modular aufgebaut ist, wobei insbesondere Module und/oder Teile von Modulen austauschbar ausgebildet sind.

11. Verfahren (24) zur Validierung eines Bestrahlungsvorgangs, insbesondere zur Validierung einer Bestrahlungsplanung, wobei der Bestrahlungsvorgang (26) an einer Bestrahlungsphantomvorrichtung (1) erfolgt, die zumindest eine Bewegungsvorrichtung (10, 15) zur Bewegung von zumindest einem ersten Teilbereich (5, 6, 7, 8, 9) eines Bestrahlungsphantoms (2) relativ zu zumindest einem zweiten Teilbereich (5, 6, 7, 8, 9, 17) des Bestrahlungsphantoms (2) aufweist, und wobei das Bestrahlungsphantom (2) zumindest bereichsweise bestrahlungsphantomvorbildkonforme Bestrahlungseigenschaften aufweist, **dadurch gekennzeichnet, dass** eine Bewegungsvorrichtung (10) eine überlagerte Bewegung zweier Einzel-Bewegungsmuster erzeugt sowie zumindest zeitweise und/oder zumindest bereichsweise zumindest eine zeitabhängige 6D-Bewegung durchführt.

12. verfahren (24) nach Anspruch 11, **dadurch gekennzeichnet, dass** zumindest zeitweise und/oder zumindest bereichsweise eine Bestrahlungsphantomvorrichtung (1) nach einem der Ansprüche 1 bis 11 verwendet wird.

13. Verfahrens (24) nach Anspruch 11 oder 12, **dadurch gekennzeichnet, dass** das Verfahren unter Verwendung von Störsignalen durchgeführt wird, welche insbesondere in einer Speichereinrichtung (13) abgelegt sind und/oder durch zumindest eine Messvorrichtung (19) gewonnen werden.

## Claims

1. Irradiation phantom device (1), in particular irradiation phantom device for validating an irradiation process (26), preferably for validating an irradiation plan, having an irradiation phantom (2) and at least one movement device (10, 15) for moving at least one first subregion (5, 6, 7, 8, 9) of the irradiation phantom (2) relative to at least one second subregion (5, 6, 7, 8, 9, 17) of the irradiation phantom (2), wherein, at least at times and/or at least in some regions, the irradiation phantom (2) has irradiation characteristics which comply with the irradiation phantom model, **characterized in that** a movement device (10) is formed in such a way that it can generate a superimposed movement of two individual movement patterns and, at least at times and/or at least in some regions, can execute at least one time-dependent 6D movement.

2. Irradiation phantom device (1) according to Claim 1, **characterized in that**, at least in some regions, the irradiation phantom (2) has treatment irradiation characteristics (3) which comply with the irradiation phantom model and/or, at least in some regions, measurement irradiation characteristics which comply with the irradiation phantom model.

3. Irradiation phantom device (1) according to Claim 1 or 2, **characterized in that**, at least in some regions, the irradiation phantom (2) has attenuation characteristics which are compliant with the irradiation phantom model and/or secondary irradiation emission characteristics which are compliant with the irradiation phantom model and/or scatter characteristics which are compliant with the irradiation phantom model, in particular for subregions (5, 6, 7, 8) of the irradiation phantom (2) which lie adjacent and/or proximally and/or axially in a disc-like manner and/or conically with respect to a target volume region (9).

4. Irradiation phantom device (1) according to one of the preceding claims, **characterized in that**, at least in some regions, the irradiation phantom (2) has reflection characteristics which are compliant with the irradiation phantom model and/or secondary irradiation emission characteristics which are compliant with the irradiation phantom model and/or scatter characteristics which are compliant with the irradiation phantom model, in particular for subregions (5, 6, 7, 8, 17) of the irradiation phantom (2) which lie adjacent and/or distally and/or axially in a disc-like manner and/or conically with respect to a target volume region (9).

5. Irradiation phantom device (1) according to one of the preceding claims, **characterized by** at least one movement transmission means (11, 16) which, at least in some regions, has irradiation characteristics which are compliant with the irradiation phantom model.

6. Irradiation phantom device (1) according to one of the preceding claims, **characterized by** at least one movement generation device (10, 15), in particular at least one electric motor device (15), at least one stepper motor (15), at least one linear motor, at least one hydraulic device, at least one pneumatic device and/or at least one robotic arm device (10).

7. Irradiation phantom device (1) according to one of the preceding claims, **characterized in that**, at least in some regions, the irradiation phantom (2) is modelled on a real, in particular biological, model and particularly preferably, at least partially and/or at least in some regions, has implant devices.

8. Irradiation phantom device (1) according to one of the preceding claims, **characterized by** at least one control device (13, 14, 19), in particular by at least one electronic control device (13, 14, 19) which preferably has at least one data storage device and/or at least one data recording device (19).

9. Irradiation phantom device (1) according to one of the preceding claims, **characterized by** at least one detector device (9, 21, 23), in particular by at least one film detector device (9, 21, 23) and/or by at least one time-resolved detector device which is preferably arranged in a replaceable manner.

10. Irradiation phantom device (1) according to one of the preceding claims, **characterized in that** it is at least partially constructed in a modular manner, wherein, in particular, modules and/or parts of modules are designed to be replaceable.

11. Method (24) for validating an irradiation process, in particular for validating an irradiation plan, wherein the irradiation process (26) is carried out on an irradiation phantom device (1) which has at least one movement device (10, 15) for moving at least one first subregion (5, 6, 7, 8, 9) of an irradiation phantom (2) relative to at least one second subregion (5, 6, 7, 8, 9, 17) of the irradiation phantom (2), and wherein, at least in some regions, the irradiation phantom (2) has irradiation characteristics which comply with the irradiation phantom model, **characterized in that** a movement device (10) generates a superimposed movement of two individual movement patterns and, at least at times and/or at least in some regions, executes at least one time-dependent 6D movement.

12. Method (24) according to Claim 11, **characterized in that**, at least at times and/or at least in some regions, an irradiation phantom device (1) according to one of Claims 1 to 11 is used.

13. Method (24) according to Claim 11 or 12, **characterized in that** the method is carried out using interference signals which in particular are stored in a storage device (13) and/or are obtained by means of at least one measuring device (19).

## Revendications

1. Dispositif fantôme d'irradiation (1), en particulier dispositif fantôme d'irradiation (1) pour la validation d'un processus d'irradiation (26), de préférence pour la validation d'un plan d'irradiation, présentant un fantôme d'irradiation (2) et au moins un dispositif de mouvement (10, 15) pour déplacer au moins un premier secteur partiel (5, 6, 7, 8, 9) du fantôme d'irradiation (2) relativement à au moins un deuxième secteur partiel (5, 6, 7, 8, 9, 17) dudit fantôme d'irradiation (2), sachant que le fantôme d'irradiation (2) présente au moins par intermittence et/ou au moins par zones des propriétés d'irradiation conformes à un modèle de fantôme d'irradiation, **caractérisé en ce qu'**un dispositif de mouvement (10) est conçu de telle manière qu'il peut générer un mouvement superposé de deux modèles de mouvement individuels ainsi qu'effectuer au moins par intermittence et/ou au moins par zones au moins un mouvement 6D en fonction du temps.

2. Dispositif fantôme d'irradiation (1) selon la revendication 1, **caractérisé en ce que** le fantôme d'irradiation (2) présente au moins par zones des propriétés de rayonnement de traitement (3) conformes à un modèle de fantôme d'irradiation et/ou au moins par zones des propriétés de rayonnement de mesure conformes à un modèle de fantôme d'irradiation.

3. Dispositif fantôme d'irradiation (1) selon la revendication 1 ou 2, **caractérisé en ce que** le fantôme d'irradiation (2) présente au moins par zones des propriétés d'atténuation conformes à un modèle de fantôme d'irradiation et/ou des propriétés d'émission de rayonnement secondaire conformes à un modèle de fantôme d'irradiation et/ou des propriétés de diffusion conformes à un modèle de fantôme d'irradiation, en particulier pour des secteurs partiels (5, 6, 7, 8) du fantôme d'irradiation (2) voisines et/ou proximales et/ou axialement discoïdes et/ou conoïdes par rapport à une zone de volume cible (9).

4. Dispositif fantôme d'irradiation (1) selon l'une des revendications précédentes, **caractérisé en ce que** le fantôme d'irradiation (2) présente au moins par zones des propriétés de réflexion conformes à un modèle de fantôme d'irradiation et/ou des propriétés d'émission de rayonnement secondaire conformes à un modèle de fantôme d'irradiation et/ou des propriétés de diffusion conformes à un modèle de fantôme d'irradiation, en particulier pour des secteurs partiels (5, 6, 7, 8, 17) du fantôme d'irradiation (2) voisines et/ou distales et/ou axialement discoïdes et/ou conoïdes par rapport à une zone de volume cible (9).

5. Dispositif fantôme d'irradiation (1) selon l'une des revendications précédentes, **caractérisé par** au moins un moyen de transmission de mouvement (11, 16) qui présente au moins par zones des propriétés d'irradiation conformes à un modèle de fantôme d'irradiation.

6. Dispositif fantôme d'irradiation (1) selon l'une des revendications précédentes, **caractérisé par** au moins un dispositif générateur de mouvement (10, 15), en particulier au moins un dispositif à moteur électrique (15), au moins un moteur pas à pas (15), au moins un moteur linéaire, au moins un dispositif hydraulique, au moins un dispositif pneumatique et/ou au moins un dispositif à bras de robot (10).

7. Dispositif fantôme d'irradiation (1) selon l'une des revendications précédentes, **caractérisé en ce que** le fantôme d'irradiation (2) est au moins par zones la reproduction d'un modèle réel, en particulier biologique, et présente en particulier de préférence au moins en partie et/ou au moins par zones des dispositifs d'implant.

8. Dispositif fantôme d'irradiation (1) selon l'une des revendications précédentes, **caractérisé par** au moins un organe de commande (13, 14, 19), en particulier par au moins un organe de commande électronique (13, 14, 19), qui présente de préférence au moins un dispositif de mémorisation de données et/ou au moins un dispositif d'enregistrement de données (19).

9. Dispositif fantôme d'irradiation (1) selon l'une des revendications précédentes, **caractérisé par** au moins un dispositif détecteur (9, 21, 23), en particulier par au moins un dispositif détecteur à film (9, 21, 23) et/ou par au moins un dispositif détecteur à résolution temporelle, lequel est disposé de préférence de manière interchangeable.

10. Dispositif fantôme d'irradiation (1) selon l'une des revendications précédentes, **caractérisé en ce que** ledit dispositif est de conception modulaire au moins en partie, sachant qu'en particulier des modules et/ou des parties de modules sont conçus de manière interchangeable.

11. Procédé (24) pour la validation d'un processus d'irradiation, en particulier pour la validation d'un plan d'irradiation, sachant que le processus d'irradiation (26) a lieu sur un dispositif fantôme d'irradiation (1) qui présente au moins un dispositif de mouvement (10, 15) pour déplacer au moins un premier secteur partiel (5, 6, 7, 8, 9) d'un fantôme d'irradiation (2) relativement à au moins un deuxième secteur partiel (5, 6, 7, 8, 9, 17) dudit fantôme d'irradiation (2), et que le fantôme d'irradiation (2) présente au moins par zones des propriétés d'irradiation conformes à un modèle de fantôme d'irradiation, **caractérisé en ce qu'**un dispositif de mouvement (10) génère un mouvement superposé de deux modèles de mouvement individuels et effectue au moins par intermittence et/ou au moins par zones au moins un mouvement 6D en fonction du temps.

12. Procédé (24) selon la revendication 11, **caractérisé en ce qu'**est mis en oeuvre au moins par intermittence et/ou au moins par zones un dispositif fantôme d'irradiation (1) selon l'une des revendications 1 à 11.

13. Procédé (24) selon la revendication 11 ou 12, **caractérisé en ce que** le procédé est réalisé en utilisant des signaux d'interférence qui sont en particulier stockés dans un dispositif de mémorisation (13) et/ou acquis par au moins un dispositif de mesure (19).
